Europäisches Patentamt

European Patent Office

Office européen des brevets

(11) Veröffentlichungsnummer: **0 518 288 A1**

## (12) EUROPÄISCHE PATENTANMELDUNG

(21) Anmeldenummer: **92109738.2**

(22) Anmeldetag: **10.06.92**

(51) Int. Cl.5: **A61F 13/15**

(30) Priorität: **10.06.91 DE 4119096**

(43) Veröffentlichungstag der Anmeldung:
**16.12.92 Patentblatt 92/51**

(84) Benannte Vertragsstaaten:
**AT BE CH DE DK FR GB GR IT LI LU NL SE**

(71) Anmelder: **FIRMA RENATE KRIEGER**
**Lange Wand 8**
**W-3280 Bad Pyrmont(DE)**

(72) Erfinder: **Krieger, Klaus**
**Kurfürstenstrasse 9a**
**W-3280 Bad Pyrmont(DE)**

(74) Vertreter: **TER MEER - MÜLLER -**
**STEINMEISTER & PARTNER**
**Mauerkircherstrasse 45**
**W-8000 München 80(DE)**

(54) **Feuchtigkeitssperrendes Textilgebilde.**

(57) Feuchtigkeitssperrendes Textilgebilde mit einer Textilbahn (12), die einseitig mit einer wasserundurchlässigen Schutzfolie (14) versehen ist, und mit einem die Schutzfolie nahezu auf seiner gesamten Oberfläche abdeckenden Polster (16) aus saugfähigem Material, dadurch gekennzeichnet, daß zwischen dem Rand des Polsters und dem Rand der Textilbahn ringsum ein Zwischenraum (20) vorgesehen ist, in dem die Schutzfolie freiliegt.

*Fig. 1*

EP 0 518 288 A1

Die Erfindung betrifft ein feuchtigkeitssperrendes Textilgebilde, beispielsweise eine Windelhose oder eine Krankenunterlage.

Angesichts der sich verschärfenden Entsorgungsprobleme bei Einweg-Windeln aus Zellstoff besteht zunehmendes Interesse an Windeln, Krankenunterlagen und dergleichen aus textilen Materialien, die waschbar und somit wiederverwendbar sind. Es sind Windelhosen dieser Art bekannt, bei denen auf eine die Außenlage bildende Textilbahn, beispielsweise aus Baumwolle, eine wasserundurchlässige Schutzfolie aus einem thermoplastischen Kunststoff aufkaschiert ist. Auf der Körperseite ist die Schutzfolie der Windel ganzflächig durch ein saugfähiges, hautfreundliches Polster abgedeckt, das beispielsweise ebenfalls aus Baumwolle besteht. Die anfallende Feuchtigkeit kann somit von dem Polster aufgesaugt werden, und die Schutzfolie verhindert, daß die Feuchtigkeit nach außen dringt. Damit auch angesichts der beim Waschen auftretenden Beanspruchungen eine ausreichende Haltbarkeit der Windelhose gewährleistet ist, muß für eine ausreichend feste Verbindung des Polsters mit dem äußeren Textilmaterial gesorgt werden. Dies wird bei der herkömmlichen Windelhose dadurch erreicht, daß das Polster und die äußere Textilbahn längs des Saumes direkt miteinander vernäht sind.

Ein Nachteil der herkömmlichen Windelhose besteht jedoch darin, daß die von dem Polster aufgenommene Feuchtigkeit durch die Perforation der Schutzfolie und die Dochtwirkung der Textillagen und des Nähgarns im Saumbereich auf die Außenseite der Schutzfolie gelangt und sich aufgrund der Dochtwirkung in der äußeren Textillage verteilt. Durch diesen Mechanismus können auf die Dauer auch relativ große Feuchtigkeitsmengen nach außen gelangen, so daß kein zufriedenstellender Nässeschutz gewährleistet ist.

Der Erfindung liegt die Aufgabe zugrunde, ein feuchtigkeitssperrendes Textilgebilde zu schaffen, bei dem die Feuchtigkeit zuverlässig in dem Polster auf der Innenseite der Schutzfolie zurückgehalten wird und nicht nach außen gelangt.

Diese Aufgabe wird erfindungsgemäß mit den in Anspruch 1 angegebenen Merkmalen gelöst. Vorteilhafte Welterbildungen der Erfindung sind in den Unteransprüchen angegeben.

Erfindungsgemäß ist das Saugpolster so auf der mit der Schutzfolie beschichteten Seite der Textilbahn angeordnet, daß zwischen dem Rand des Polsters und dem Saum der Textilbahn ringsum ein Randstreifen der Schutzfolie freibleibt. Die Breite des Randstreifens liegt beispielsweise in der Größenordnung von 1 cm und ist so bemessen, daß die Bildung von Feuchtigkeitsbrücken zuverlässig vermieden wird. Andererseits kann die Breite dieses Randstreifens jedoch so gering gehalten werden, daß die Hautatmung nicht in größeren Flächenbereichen blockiert wird und es nicht zu einer unangenehmen Hautreizung im Bereich des Randstreifens kommt.

In einer bevorzugten Ausführungsform wird das Polster durch eine ein- oder mehrlagige Textilbahn gebildet, die ebenso wie die äußere Textillage mit einer Schutzfolie aus thermoplastischem Kunststoff kaschiert ist, und die Schutzfolien der äußeren Textillage und des Polsters sind auf ganzer Fläche miteinander verschweißt. Auf diese Weise wird eine haltbare Verbindung zwischen dem Polster und der äußeren Textilbahn hergestellt, ohne daß die Schutzfolie durch Nähte oder dergleichen perforiert wird. Die Verdoppelung der Schutzfolie im Bereich des Polsters hat zudem den Vorteil, daß der Nässeschutz auch dann noch nach häufigem Waschen aufrechterhalten werden kann, wenn eine einzelne Lage der Schutzfolie bereits spröde oder rissig geworden wäre.

Das erfindungsgemäße Textilgebilde zeichnet sich somit durch eine hohe Haltbarkeit und einen entsprechend hohen Wiederverwendungsgrad aus und gestattet es, anfallende Feuchtigkeit zuverlässig zurückzuhalten.

Im folgenden wird ein bevorzugtes Ausführungsbeispiel der Erfindung anhand der Zeichnungen näher erläutert.

Es zeigen:

Fig. 1     einen schematischen Schnitt durch einen Zuschnitt für eine Windelhose; und

Fig. 2     den Zuschnitt gemäß Figur 1 in der Draufsicht.

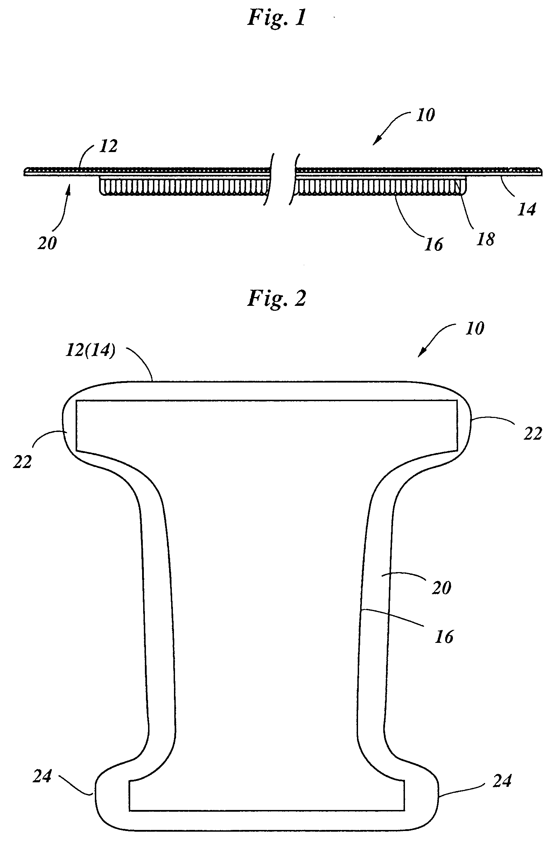

Wie in Figur 1 zu erkennen Ist, wird ein Zuschnitt 10 für eine Windelhose durch eine Textilbahn 12, beispielsweise ein Baumwollgewebe, gebildet, das auf einer Seite, unten in Figur 1, mit einer wasserundurchlässigen Schutzfolie 14 aus thermoplastischem Kunststoff kaschiert ist. Die Schutzfolie 14, die die Innenseite der Windelhose bildet, ist großflächig mit einem Polster 16 aus saugfähigem Textilmaterial bedeckt. Das Polster 16 wird beispielsweise durch einen Baumwoll-Frottierstoff gebildet und ist ebenfalls einseitig mit einer Schutzfolie 18 aus thermoplastischem Kunststoff kaschiert. Wahlweise kann das Polster 16 auch aus mehreren Textillagen bestehen, die längs des Saumes miteinander vernäht sind und von denen die oberste Lage die Schutzfolie 18 aufweist.

Der Zuschnitt des Polsters 16 Ist deutlich Kleiner als der Zuschnitt der Textilbahn 12 und ist so auf der Textilbahn angebracht, daß ringsum ein Randstreifen 20 gebildet wird, in dem die Oberfläche der Schutzfolie 14 freiliegt. Der Randstreifen hat bei dem Zuschnitt 10 eine Breite von etwa 2 bis 3 cm. Nach dem Umsäumen der Textilbahn 14 verbleibt somit noch ein Streifen mit einer Breite von etwa 1,5 cm, der den Rand des Polsters 16 vom Saum der Textilbahn 12 trennt. Durch diesen Randstreifen ist sichergestellt, daß zwischen dem Polster 16 und der Textilbahn 12 keine kapillaren Feuchtigkeitsbrücken bestehen, über die die von dem Polster 16 aufgesaugte Flüssigkeit in die äußere Textilbahn 12 eindringen kann. Durch die Säume der Textilbahn 12 und des Polsters 16 wird eine flächige Berührung des Randstreifens 20 mit der Haut weitgehend vermieden.

Bei der Herstellung werden die mit der Schutzfolie 14 kaschierte Textilbahn 12 und das mit der Schutzfolie 18 kaschierte Polster 16 so aufeinandergelegt, daß die Schutzfolien 14 und 18 einander zugewandt sind. Die Schutzfolien werden dann durch Wärme- und Druckeinwirkung ganzflächig miteinander verschweißt. Auf diese Weise wird eine haltbare Verbindung zwischen der Textilbahn 12 und dem Polster 16 geschaffen. Da die Schutzfolie 14 nicht durch Nähte perforiert wird, kann die Feuchtigkeit auch nicht durch die Nahtstiche und durch die Kapillarwirkung des Nähgarns in die äußere Textilbahn 12 gelangen.

In einer modifizierten Ausführungsform werden die Schutzfolien 14 und 18 lediglich am Rand miteinander verklebt oder verschweißt, damit das Material einen weicheren Griff behält. Durch die Verklebung oder Verschweißung in den Randbereichen wird sichergestellt, daß das Polster 16 nicht in Bezug auf die Textilbahn 12 verrutscht, so daß der als Feuchtigkeitssperre dienende Randstreifen 20 dauerhaft erhalten bleibt.

Wie aus Figur 2 hervorgeht, ist das Polster 16 auf seinem gesamten Umfang von dem Randstreifen 20 umgeben. Lediglich im Bereich der oberen seitlichen Verschlußteile 22 reicht das Polster 16 bis unmittelbar an den Saum der Textilbahn 12 heran. In diesem Bereich werden Klettverschlüsse aufgenäht, die beim Schließen der Windelhose mit der äußeren Textilbahn 12 an den unteren seitlichen Verschlußteilen 24 in Kletteingriff gebracht werden. In der Gebrauchsstellung liegen somit die oberen Verschlußteile 22 außen auf den unteren Verschlußteilen 24 auf, so daß durch die unteren Verschlußteile 24 eine wirksame Feuchtigkeitssperre gebildet wird und die Dichtheit der Windelhose auch dann nicht beeinträchtigt wird, wenn im Bereich der oberen Verschlußteile 22 eine Textilbrücke zwischen dem Polster 16 und der äußeren Textillage 12 besteht.

Die Erfindung, die hier am Beispiel einer Windelhose erläutert wurde, ist nicht auf diesen speziellen Anwendungsfall beschränkt, sondern ist bei allen Arten von Einlagen oder Unterlagen anwendbar, bei denen ein Feuchtigkeitsübertritt von einer Seite zur anderen verhindert werden soll.

**Patentansprüche**

1. Feuchtigkeitssperrendes Textilgebilde mit einer Textilbahn (12), die einseitig mit einer wasserundurchlässigen Schutzfolie (14) versehen ist, und mit einem die Schutzfolie nahezu auf seiner gesamten Oberfläche abdeckenden Polster (16) aus saugfähigem Material, dadurch **gekennzeichnet,** daß zwischen dem Rand des Polsters (16) und dem Rand der Textilbahn (12) ringsum ein Zwischenraum (20) vorgesehen ist, in dem die Schutzfolie (14) freiliegt.

2. Textilgebilde nach Anspruch 1, dadurch **gekennzeichnet,** daß das Polster (16) durch Befestigungstechniken wie Kleben oder Schweißen ohne Nähte auf der Schutzfolie (14) befestigt ist.

3. Textilgebilde nach Anspruch 2, dadurch **gekennzeichnet,** daß sowohl die Textilbahn (12) als auch das Polster (16) einseitig mit einer Schutzfolie (14,18) aus thermoplastischem Material versehen sind und daß die Schutzfolien der Textilbahn und des Polsters miteinander verschweißt sind.

**Fig. 1**

**Fig. 2**

Europäisches Patentamt

# EUROPÄISCHER RECHERCHENBERICHT

Nummer der Anmeldung

EP 92 10 9738

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X | US-A-5 009 652 (C.I.MORGAN AND M.M.JUDY)<br>* Spalte 1, Zeile 39 - Spalte 2, Zeile 53 *<br>* Spalte 4, Zeile 27 - Zeile 37 *<br>* Spalte 9, Zeile 8 - Zeile 18 *<br>* Spalte 9, Zeile 66 - Zeile 67 *<br>* Spalte 10, Zeile 34 - Zeile 40 *<br>* Spalte 10, Zeile 48 - Zeile 50 *<br>* Spalte 11, Zeile 24 - Zeile 27; Abbildungen 1-2 *<br>--- | 1-2 | A61F13/15 |
| A | FR-A-2 127 466 (D.MAAREK)<br>* Seite 2, Zeile 19 - Seite 3, Zeile 9; Abbildungen 1,3 *<br>--- | 1-3 | |
| A | US-A-3 779 246 (F.K.MESEK AND V.L.REPKE)<br>* Zusammenfassung *<br>* Seite 5, Zeile 23 - Zeile 26; Abbildungen 1-2 *<br>*<br>----- | 1-2 | |
| | | | RECHERCHIERTE SACHGEBIETE (Int. Cl.5 )<br><br>A61F |

Der vorliegende Recherchenbericht wurde für alle Patentansprüche erstellt

| Recherchenort | Abschlußdatum der Recherche | Prüfer |
|---|---|---|
| DEN HAAG | 19 AUGUST 1992 | NICE P. |